Europäisches Patentamt

European Patent Office

Office européen des brevets

(19)

(11) Veröffentlichungsnummer: **0 277 233 B1**

(12) **EUROPÄISCHE PATENTSCHRIFT**

(45) Veröffentlichungstag der Patentschrift: 04.04.90

(21) Anmeldenummer: 86906502.9

(22) Anmeldetag: 11.08.86

(86) Internationale Anmeldenummer:
PCT/SU86/00077

(87) Internationale Veröffentlichungsnummer:
WO 88/01218 25.02.88 Gazette 88/05

(51) Int. Cl.⁵: **B 23 K 31/10, B 23 K 9/16,**
**A 61 B 17/32**

(54) **VORRICHTUNG ZUM SCHNEIDEN VON BIOLOGISCHEN GEWEBEN MIT PLASMABÖGEN.**

(43) Veröffentlichungstag der Anmeldung:
10.08.88 Patentblatt 88/32

(45) Bekanntmachung des Hinweises auf die
Patenterteilung:
04.04.90 Patentblatt 90/14

(84) Benannte Vertragsstaaten:
DE FR GB IT

(56) Entgegenhaltungen:
FR-A-2 450 659
US-A-3 434 476
US-A-3 674 978
US-A-3 838 242
US-A-3 991 764
US-A-4 250 373
US-A-4 369 919

(73) Patentinhaber: 2-i MOSKOVSKY
GOSUDARSTVENNY MEDITSINSKY INSTITUT
IMENI N.I. PIROGOVA
ul. Ostrovityanova 1
Moscow, 117437 (SU)

(72) Erfinder: BERESNEV, Alexei Sergeevich
ul. Ostrovskogo, 6-59
Smolensk, 214006 (SU)
Erfinder: SERYKH, Leonid Afanasievich
ul. Oktyabrskoi Revoljutsii, 7-106
Smolensk, 214000 (SU)
Erfinder: SAVELIEV, Viktor Sergeevich
ul. Donskaya, 27-29
Moscow, 117049 (SU)
Erfinder: VOLKOEDOV, Valery Sergeevich
ul. Marshala Ustinova, 16-2-908
Moscow, 121360 (SU)
Erfinder: STUPIN, Igor Viktorovich
ul. Akademika Bakuleva, 4-122
Moscow, 117513 (SU)

(74) Vertreter: Ebbinghaus, Dieter et al
v. FÜNER, EBBINGHAUS, FINCK Postfach 95 01
60
D-8000 München 95 (DE)

Courier Press, Leamington Spa, England.

EP 0 277 233 B1

## Beschreibung

Technisches Gebiet

Die Erfindung bezieht sich auf medizinische Geräte und betrifft insbesondere Vorrichtungen zum Plasma-Lichtbogenschnieden von biologischen Geweben.

Zurgrundeliegender Stand der Technik

Um biologische Gewebe zu schneiden, können Vorrichtungen eingesetzt werden, bei welchen ein indirekter Lichtbogen oder ein Mikroplasmastrahl erzeugt wird.

Bekannt ist eine Vorrichtung zum Plasma-Lichtbogenschneiden, die ein Gehäuse enthält, in welchem eine Katoden- und eine Anodeneinheit aufgenommen sind. In der Katodeneinheit ist eine Elektrode befestigt, die einen Kegelbereich mit einem spitzigen Ende aufweist. Die Anodeneinheit enthält eine Düse komplizierter Form, in welcher die Elektrode untergebracht ist. Ihr spitziges Ende ist zur Stelle der Bogenbildung hin gerichtet. Die Katodeneinheit weist Kanäle zur Plasmagaszuführung auf. (s. z.B. Elektrodugovye plazmatrony/Lichtbogenplasmabrenner/Reklameschrift, Nowosibirsk, 1980, S. 34 bis 35).

Diese Vorrichtungen sind ungeeignet, biologische Gewebe zu schneiden, da sie eine geringe Leistung des Mikroplasmastrahls besitzen, die nicht ausrichend ist, um beim Schneiden biologischer Gewebe erforderliche Geschwindigkeit zu erreichen; darüber hinaus wird bei ihnen ein hoher Betriebsdruck das Plasmagases verwendet, wo durch die biologischen Gewebe mit gas übersättigt werden. Die Temperatur des mit der bekannten Vorrichtung erzeugten Plasmastrahls überschreitet 5 000 °C nicht, was zum Schneiden biologischer Gewebe nicht ausreicht und thermische Verletzung der biologischen Gewebe an deren Schnitträndern in große Tiefe verursacht.

Bekannt ist auch eine Vorrichtung zum Plasma-Lichtbogenschneiden von biologischen Geweben, die eine Katoden- und eine Anodeneinheit enthält. Die Elektrode der Katodeneinheit weist einen Kegelbereich auf, der mit dem spitzigen Ende der Stelle der Bogenzündung zugewandt ist. In der Katodeneinheit sind Kanäle zur Plasmagaszuführung eingearbeitet. Die Anodeneinheit weist eine Düse auf, die den Kegelbereich der Elektrode aufnimmt. Die Düse besteht aus einer Zyloinderkammer, einer kegelförmigen Kammer, einer Brennkammer und einer Kammer zur Plasmabogenbildung die in der Reihenfolge ihrer Aufzählung angeordnet sind (s. z.B. SU-Urheberscheinschrift 275261, Amtsbaltt Nr. 22, 1970, A. S. Beresnev u.a.).

Bei dieser Vorrichtung brennt der Lichtbogen in der Brennkammer bei geringem Verbrauch bzw. Druck des Plasmagases unstabil, was verbessert werden muß, damit die genannte Vorrichtung in der Medizin verwendet werden könnte. Der Anodenfleck wandert dabei aus der Brennkammer in die kegelförmige Kammer der Düse über, wodurch die Spannung am Bogen und seine Leistung verringert sowie die Schnitt und die Koagulationseigenschaften des Plasmastrahls beeinträchtigt werden. Um den Anodenfleck in die Brennkammer wieder einzuleiten, muß der Betriebsdruck des Plasmagases z.B. auf 2.10⁵ Pa erhöht und dann auf den Arbeitsdruck von 1.10⁵ Pa nochmals herumtergebracht werden. Durch diese periodische Gas-Betriebsdruckerhöhung wird eine vergrösserte Ausströmgeschwindigkeit des Plasmastrahls bewirkt, die Arbeit des Chirurgen erschwert und die Wahrscheinlichkeit erhöht, daß nicht opperierte Organe verletzt werden.

Offenbargung der Erfindung

Der Erfindung wurde die Aufgabe zugrundegelegt, eine Vorrichtung zum Plasma-Lichtbogenschneiden von biologischen Geweben zu schaffen, bei welcher durch Änderung der Konstruktion der Anodeneinheit ein stabiles Brennen des Lichtbogens bei geringem Plasmagasverbrauch bzw. -druck erreicht wäre.

Diese Aufgabe wird dadurch gelöst, daß bei einer Vorrichtung zum Plasma-Lichtbogenschneiden von biologischen Geweben, in welcher eine Katodeneinheit ein Gehäuse mit Kanälen zur Plasmagaszuführung und eine im Gehäuse aufgenommene und einen Kegelbereich aufweisende Elektrode enthält, während eine Anodeneinheit ein Gehäuse und eine Düse besitzt, in welcher der der Stelle der Plasmastrahlbildung zugewandte Kegelbereich der Elektrode untergebracht ist und welche nacheinander angeordnete eine Zyinderkammer, eine kegelförmige Kammer, eine Brennkammer und eine Kammer zur Plasmastrahlbildung aufweist, erfindungsgemäß der Kegelbereich der Elektrode die Form eines Kegelstumpfes aufweist, dessen kleinere Grundfläche, welche die Stirnfläche der Elektrode darstellt, der Stelle der Plasmastrahlbildung zugewandt ist, und die Elektrode in der Zylinder- und der kegelförmigen Kammer so angebracht ist, daß der Abstand von der Brennkammer zur Stirnfläched der Elektrode gleich 0,1 bis 0,5 der Länge der kegelförmigen Kammer ist, während die länge des Kegelbereiches der Elektrode 3,5 bis 4,5 Elektrodendurchmesser beträgt.

Zweckmäßig ist, daß die Plasmagaszufuhrkanäle der Katodeneinheit eine Länge aufweist, die der Länge der Kammer zur Plasmastrahlbildung in der Düse gleich ist, und von der Stirnfläche der Elektrode um die Länge deren Kegelbereiches entfernt wären.

Kurze Beschreibung der Zeichnungen

Im weiteren wird die Erfindung durch die Beschreibung ihres Ausführungsbeispiels unter Bezugnahme auf die beiliegende Zeichnungen näher erläutert, in welchen es zeigen:

Fig. 1 Gesamtansicht einer erfindungsgemäßen Vorrichtung zum Plasma-Lichtbogenschneiden von biologischen Geweben im Längsschnitt;

Fig. 2 die gleiche Vorrichtung im Schnitt nachu Linie II—II in Fig. 1.

Bevorzugte Ausführungsform der Erfindung

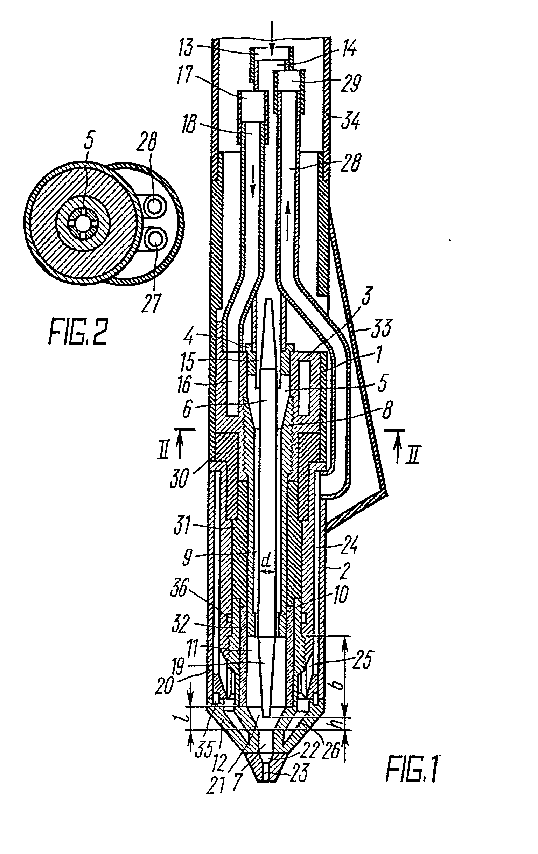

die Vorrichtung zum Plasma-Lichtbogenschneiden von biologischen Geweben enthält eine Katodeneinheit 1 (Fig. 1) und eine Anodeneinheit 2. Die Katodeneinheit 1 enthält ein Gehäuse 3, in welchem eine Zange 4 mit Einschnitten 5 eingepreßt ist, die Gaskanäle darstellen. In der Zange 4 ist eine Elektrode 6 eingesetzt, die in der erforderlichen Lage inbezug auf die Oberkante einer Brennkammer 7 durch eine Ganzzange 8 gesichert ist.

Die Zange 8 weist Gaskanäle 9 und 10 zwecks Zuführung das Plasmagases in die zylindrische Kammer 1 einer Düse 12 auf.

Die Katodeneinheit 1 weist einen Gasstutzen 13 auf, der über ein Rohr 14 mit Zufuhrkanälen 15 für das Plasmagas in der Zange 4 verbunden ist.

Die Katodeneinheit 1 weist darüber hinaus noch einen Kanal 16 zur Kühlmittelzuführung über einen Stutzen 17 und ein Rohr 18. auf. Die Elektrode 6 besitztt einen kegelstumpfförmigen Kegelbereich 19. Die kleinere Grundfläche des Kegelstumpfes stellt die Stirnfläche der Elektrode 6 dar.

Die Anodeneinheit 2 enthält ein Gehäuse 20 und eine Düse 12, deren innerer Teil die zylindrische Gaskammer 11, eine kegelförmige Kammer 21 und einen plasmabildenden Kanal 22 aufweist, der aus der Brennkammer 7 und einer Kammer 23 zur Bildung des Plasmastrahls besteht.

Die Anodeneinheit 2 wird durch Kühlmittelzuführung über ihre Kanäle 24 und 25 sowie über Kanäle 26 un die düse 12 herum abgekühlt. Das Kühlmittel wird über einen Stutzen 27 (Fig. 2) zu- und überen Rohr 28 und einen Stutzen 29 (Fig. 1) abgeleitet.

Die Einheiten 1 und 2 sind durch Isolatoren 30, 31, 32,33 und 34 elektrisch isoliert.

Die Düse 12 weist einen Gewindebereich auf, mittels welches sie mit der Anodeneinheit 2 verbunden wird. Im Stirnbereich der Anodeneinheit 2 und in deren innerem Bereich sind Rillen durchgedreht, in welchen Dichtungen 35 und 36 angeordnet sind. Die Querschnitte dieser Gummidichtungen sind so bemessen, daß sie ein leichtes vollständiges Hineinschrauben der Düse 12 und die Dichtheit der Vorrichtung gegen Kühlmitteleindringung bei einem Mindestdruck von 2,53 Bar (2,5 atm) gewährleisten.

Der untere Stirnbereich der Zange 8 ist so ausgebildet, daß er erlaubt, beim Einschrauben der Zange 8 in das Gehäuse 3 er Katodeneinheit 1 die Elektrode 6 in der kegelförmigen Kammer 21 der Düse 12 inbezug auf die obere Stirnseite der Brennkammer 7 zu sichern. Die Elektrode 6 ist dabei mit ihrem Kegelbereich 19 in den Kammern 11 und 21 angeordnet und mit der Stirnfläche der Bildungsstelle des Plasmastrahls zugewandt. Der Abstand "h" von der Brennkammer 7 zur Stirnfläche der Elektrode 6 ist gleich 0,1 bis 0,5 der Länge "1" der kegelförmigen Kammer 21 gewählt. Dieser Abstand darf die Größe 0,1 l nicht unterschreiten, da sonst der Bogen unstabil brennt un die thermische Beständigkeit der Elektrode sinkt, während bei h > 0,5 l der Bogen zur bei sehr großem Plasmagasverbrauch brennt.

Die Länge "b" des Kegelbereiches 19 der Elektrode 6 wurde gleich 3,5 bis 4,5 ihres Durchmessers "d" gewählt und sie darf die Größe 3,5d nicht unterschreiten, da sonst der Bogen weniger stabil brennen wird, während bei der Länge b 4,5d der Bogen nur bei sehr großem Plasmagasverbrauch stabil brennt.

Als Kühlmittel wird bei der Vorrichtung fließendes Wasser unter einem Druck nicht unter 0,6 Bar (0,6 atm) und nicht über 1,5 Bar (1,5 atm) verwendet. Die Anwendung eines Betriebsdruckes unter 0,6 Bar (0,6 atm) und über 1,5 Bar (1,5 atm) wäre unzweckmäßig, da im ersteren Fall (unter 0,6 atm) die Kühlung der Düse 12 nicht ausreichend ist und der Bogen unstabil brennt, während beim Druck über 1,5 atm die Druckbeaufschlagung der Dichtungen 35, 36 zunimmt und folglich deren Dichtheit beeinträchtige werden kann. Wird die Dichtung 36 unhermetisch, drängt as Wasser ins Innere der Gaskammer 11 ein, wobei die elektrische Isolation der Elektrode 6 inbezug auf die Anodeneinheit 2 gestört wird. In diesem Fall ist ein elektrischer Durchschlag möglich, wenn der elektrische Bogen zwischen dem Seitenteil der Elektrode 6 und dem Gehäuse 20 der Einheit 2 entsteht.

Die Vorrichtung zum Plasma-Lichtbogenschneiden von biologischen Geweben funktioniert folgenderweise.

Zunächst wird die Vorrichtung eingerichtet, indem man die Intaktheit der Düse 12 überprüft und die Elektrode 6 mit der Zange 8 in der Kammer 21 mit erforderlichem Abstand einstellt, der etwa 0,4 bis 0,5 mm von der Offnung der Brennkammer 7 beträgt.

Es wird die vorgegebene Größe des Plasmagasverbrauchs eingestellt, die Kühlmittelzuführung eingeschaltet und die Bogenspeisespannung angeschlossen.

Das Kühlmittel wird über den Stutzen 29 und das Rohr 28 den Kanälen 24, 25 und 26 zugeführt, indem es die Anodeneinheit 2 und ihre Düse 12 abkühlt, es strömt gleichzeitig aus dem Kanal 24 über den Stutzen 27 in den Kanal 9, kühlt die Elektrode 6 und die Katodeneinheit 1 ab und wird aus der Vorrichtung über den Stutzen 17 abgeleitet.

Das Plasmagas wird dem Stutzen 13 des Rohres 18 zugeführt und über die Einschnitte 5 und den Kanal 9 in die zylindrische Gaskammer 11 der Düse 12 eingeleitet. Das stabile Brennen des Bogens in der Brennkammer 7 über ihre gesamte Oberfläche bei geringen Gas-Betriebsdrücken (bis 0,1 atm) und geringem Gasverbauch wird durch optimale Verhältnisse der Länge ihres Kegelbereichs zu ihrem Durchmesser, die Anordnung der Elektrode in der kegelfömigen Kammer der Düse inbezug auf die Brennkammer, die Kegelneigungsgröße der kegelfömigen Kammer 21, den Abstand der Gaskanäle 10 zur Brennkammer 7 und die Länge dieser Kanäle 10 in Abhängigkeit von der Länge der Kammer 23 zur Bildung des Plasmastrahls bedingt.

Nach der Einschaltung der Kühlmittel- und Plasmagazuführung wird die Gasstrecke zwischen der Stirnfläche der Elektrode 6 und der Zylinderfläche der Brennkammer 7 in der Düse 12 durchgeschlagen und ionisiert. Gleichzeitig damit wird der Elektrode 6 und der Düse 12 die Spannung von der Kraftquelle zur Speisung des eingeschnürten Bogens zugeführt. Zunächst wird der Bogen bei geringem Strom und geringem Gasverbrauch angezündet. Das ist erforderlich, um das Verspritzen im Zeitpunkt der Bogeneinschaltung zu vermeiden und die Anodeneinheit 2 dadurchzuhitzen. Dann wird der Bogenstrom auf den Arbeitsstrom erhöht und durch Einstellen das Plasmagasverbrauchs der Plasmastrahl mit erforderlichen Parametern erzeugt.

Der erfindungsgemäß vorgeschlagene Brenneraufbau erlaubt, die biologischen Gewebe wirkungsvoll und sicher unter sehr niedrigen Betriebsdrücken und kleinem Gasverbrauch zu schneiden, wodurch die Ausströmgeschwindigkeit des Gasstrahls wesentlich verringert, sein Einstellbereich erweitert wird.

Industrielle Anwendbarkeit

Die Erfindung ist für chirurgische Operationen anwendbar, z.B. um Tumore zu exzidieren und Blutgefäße an den Schnitträndern zu koagulieren.

**Patentansprüche**

1. Vorrichtung zum Plasma-Lichtbogenschneiden von biologischen Geweben, bei welcher eine Katodeneinheit (1) ein Gehäuse (3) mit Kanälen (15) zur Plasmagaszuführung und eine im Gehäuse (3) aufgenommene und einen Kegelbereich (19) aufweisende Elektrode (6) enthält, während eine Anodeneinheit (2) ein Gehäuse (20) und eine Düse (12) besitzt, in welcher ein der Stelle der Plasmastrahlbildung zugewandter Kegelbereich (19) der Elektrode (6) untergebracht ist und welche, nacheinander angeordnet, eine Zylinderkammer (11), eine kegelförmige Kammer (21), eine Brennkammer (7) und eine Kammer (23) zur Plasmastrahlbildung aufweist, dadurch gekennzeichnet, daß der Kegelbereich (19) der Elektrode (6) die Form eines Kegelstumpfes aufweist, dessen die Stirnfläche der Elektrode (6) darstellende kleinere Grundfläche der Stelle der Plasmastrahlbildung zugewandt ist, und die Elektrode (6) in der Zylinder- und der kegelförmigen Kammer (11 und 21) so angebracht ist, daß der Abstand von der Brennkanner (7) zur Stirnfläche der elektrode (6) gleich 0,1 bis 0,5 der Länge der kegelförmigen Kammer (21) ist, während die Länge des Kegelbereiches (19) der Elektrode (6) 3,5 bis 4,5 Durchmesser der Elektrode (6) beträgt.

2. Vorrichtung nach Anspruch 1, dadurch gekennzeichnet, daß die Plasmagaszufuhrkanäle (10) der Katodeneinheit (1) eine Länge aufweisen, die der Länge der Kammer (23) zur Plasmastrahlbildung in der Düse (12) gleich ist, und von der Stirnfläche der Elektrode (6) um die Länge deren Kegelbereiches (19) entfernt sind.

**Revendications**

1. Dispositif pour la coupe à l'arc de plasma de tissus biologiques, où une unité de cathode (1) contient un boîtier (3) avec des canaux (15) pour l'alimentation en gaz de plasma et une électrode (6) reçue dans le boîtier (3) et présentant une zone conique (19), tandis qu'une unité d'anode (2) possède un boîtier (20) et une tuyère (12), où est reçue une zone conique de l'électrode (6) tournée vers le point de formation du jet de plasma et qui présente, les uns à la suite des autres, une chambre cylindrique (11), une chambre conique (21), une chambre de combustion (7) et une chambre (23) pour la formation du jet de plasma, caractérisé en ce que la zone conique (19) de l'électrode (6) présente la forme d'un tronc de cone dont la plus petite surface de base représentant la surface frontale de l'électrode (6) est tournée vers le point de formation du jet de plasma et l'électrode (6) est disposée dans la chambre cylindrique et conique (11, 21) de manière que la distance de la chambre de combustion (7) à la surface frontale de l'électrode (6) soit égale à 0,1—0,5 fois la longueuer de la chambre conique (21), tandis que la longueur de la zone conique (19) de l'électrode (6) représente 3,5 à 4,5 fois le diamètre de l'électrode (6).

2. Dispositif selon la revendication 1, caractérisé en ce que les canaux d'alimentation en gaz de plasma (10) de l'unité de cathode (1) présentent une longueur qui est égale à la longueur de la chambre (23) pour la formation du jet de plasma dans la tuyère (12) et ils sont éloignés de la surface frontale de l'électrode (6) de la longueur de la zone conique (19).

**Claims**

1. A device for the plasma-arc cutting of biological tissues, in which a cathode unit (1) contains a housing (3) with ducts (15) for supplying plasma gas, and contains an electrode (6) received in the housing (3) and having a cone region (19), while an anode unit (2) has a housing (20) and a nozzle (12) which accommodates a cone region (19) of the electrode (6), which region faces the place of plasma ray formation, and which nozzle has, arranged in succession, a cylindrical chamber (11), a cone-shaped chamber (21), a combustion chamber (7) and a chamber (23) for plasma ray formation, characterised in that the cone region (19) of the electrode (6) has the shape of a truncated cone whose smaller base surface, representing the front end of the electrode (6) faces the place of plasma ray formation, and the electrode (6) is arranged in the cylindrical and the cone-shaped chamber (11 and 21) in such a way that the distance from the combustion chamber (7) to the front end of the electrode (6) equals 0.1 to 0.5 of the length of the cone-shaped chamber (21), while the length of the cone region (19) of the electrode (6) is 3.5 to 4.5 diameter of the electrode (6).

2. A device according to Claim 1, characterised in that the plasma gas supply ducts (10) of the cathode unit (1) have a length equal to the length of the chamber (23) for plasma ray formation in the nozzle (12), and are spaced from the front end of the electrode (6) at a distance amounting to the length of the cone region (19) thereof.

FIG. 2

FIG. 1